# FASCICULE DE BREVET EUROPEEN

(11) **EP 2 694 617 B1**
(45) Date de publication et mention de la délivrance du brevet: **05.06.2019**
(21) Numéro de dépôt: 12711912.1
(22) Date de dépôt: 05.04.2012
(51) Int. Cl.: C09K 8/34, C10M 105/04, C09D 11/02, C11D 3/18, A61K 8/30, A61K 31/00, C09D 5/34

(54) **COMPOSITION DE FLUIDE SPECIAL ET UTILISATION**
SPEZIELLE FLÜSSIGKEITSZUSAMMENSETZUNG UND IHRE VERWENDUNG
SPECIAL FLUID COMPOSITION AND USE THEREOF

(30) Priorité: 06.04.2011 FR 1153005
(43) Date de publication de la demande: 12.02.2014
(73) Titulaire: Total Marketing Services, 92800 Puteaux (FR)
(72) Inventeur: GERMANAUD, Laurent, F-38540 Heyrieux (FR); LAMRANI-KERN, Samia, F-94170 Le Perreux sur Marne (FR)
(74) Mandataire: Hirsch & Associés
(86) Numéro de dépôt international: PCT/EP2012/056354
(87) Numéro de publication internationale: WO 2012/136806

(56) Documents cités:
- EP-A1- 2 062 584
- EP-A2- 1 946 743
- EP-B1- 1 077 675
- WO-A2-2008/024488
- GB-A- 2 287 265
- US-A1- 2004 102 351
- US-B1- 7 592 295
- US-B1- 7 691 792

## Description

### DOMAINE TECHNIQUE

La présente invention concerne une composition de fluide spécial comprenant des poly-terpènes hydrogénés en mélange avec des fluides spéciaux d'origine pétrolière et/ou de synthèse pour des applications pétrolières, pour la construction, l'imprimerie, l'agroalimentaire, la pharmacie, l'automobile, les lubrifiants industriels, les combustibles domestiques, les applications de fluides autorisés pour les contacts alimentaires et les cosmétiques.

### ART ANTERIEUR

On appelle fluides spéciaux des liquides utilisés comme fluides industriels, fluides agricoles et horticole (l'horticulture maraîchère (ou le maraîchage) pour la production des légumes, l'arboriculture fruitière, pour la production de fruits ; la floriculture pour la production de plantes ornementales; la pépinière pour la production d'espèces ligneuses, arbres et arbustes d'ornement ou non ; la serriculture pour la production floricole et de pépinière en serre), et fluides à usage domestique obtenus généralement à partir d'hydrocarbures fossiles transformés par voies de raffinage mais aussi à partir de nombreux produits issus de la polymérisation ou l'oligomérisation d'oléfines de 3 à 4 carbones, et également d'hydrocarbures de synthèse résultant de la transformation du gaz naturel ou du gaz de synthèse issu de la biomasse et/ou du charbon. Parmi ceux-ci, on trouve des fluides de forage, des lubrifiants pour l'industrie, des fluides pour formulations destinées à l'automobile, des produits phytosanitaires, des fluides de base pour formulations des encres, des combustibles pour applications domestiques, des huiles d'extension pour les mastics, des abaisseurs de viscosité pour formulations à base de résine, des compositions pharmaceutiques et des compositions pour contact alimentaire, des fluides destinés aux formulations cosmétiques.

La nature chimique et la composition des fluides connus de l'homme du métier varient considérablement selon l'application envisagée.

Certains fluides spéciaux sont des produits obtenus à partir de raffinage de pétrole brut, dont les propriétés sont adaptées à l'application envisagée. Ainsi, on détermine l'intervalle de distillation (mesuré par l'ASTM D86 ou l'ASTM D1160 selon le choix du point final de distillation inférieur ou supérieur à 365°C), le point d'écoulement (mesuré par l'ASTM D97), la viscosité à 20 ou à 40°C (mesurée par l'ASTM D445), la densité à 15°C (mesurée par l'ASTM D4052), la teneur en soufre (mesurée par ASTM D5453), la teneur en aromatiques (mesurée par UV pour les faibles teneurs ou par HPLC IP391 pour les plus fortes teneurs), le point d'aniline (mesuré par ASTM D611) et le point éclair (mesuré par l'ASTM D93). Ces propriétés et le mode de production de ces fluides spéciaux à partir d'hydrocarbures, notamment distillés en coupes d'hydrocarbures d'origine pétrolière, constituent des caractéristiques importantes à considérer pour les adapter à différentes applications envisagées. Plus particulièrement, ces fluides spéciaux sont obtenus par hydrogénation de coupes de distillation variant de 100 à 400°C, obtenues par hydrocraquage, par hydrotraitement, par craquage catalytique, par cokage, par viscoréduction et/ou par hydrodéparaffinage. Ces fluides spéciaux sont constitués majoritairement d'isoparaffines et de naphtènes saturés, et ont une teneur en soufre inférieure à 10 ppm ainsi qu'une teneur en aromatiques inférieure à 100 ppm.

Pour ces diverses applications, on choisit souvent des gammes de points d'ébullition étroites entre le Point initial de distillation (IBP) et le Point final de distillation (FBP), l'étroitesse de la coupe permettant de contrôler la sélectivité des caractéristiques physico chimiques, et notamment :
- un point éclair ajusté, paramètre important pour satisfaire les exigences en matière de sécurité,
- une plage de viscosité plus étroite pour une mise en oeuvre plus aisée,
- des caractéristiques d'évaporation parfaitement adaptées aux applications nécessitant une étape de séchage d'une durée contrôlée,
- une valeur de tension de surface définie pour certaines applications nécessitant un contact avec des matériaux et supports (métaux, textiles, bois....)
- un pouvoir solvant élevé défini par la valeur du point d'aniline.

L'obtention de coupes étroites n'est pas toujours évidente surtout pour la reproduction de coupes de qualité identique, vendues pour une même application car cela requiert un accès à des ressources de compositions relativement constantes et à des réglages d'unités d'hydrogénation précis.

D'autres fluides spéciaux d'origine synthétique sont issus de la polymérisation et/ou de l'oligomérisation d'oléfines de 2 à 4 carbones, cette polymérisation conduisant à des produits comprenant de 2 à 5 motifs oléfiniques par chaîne. Ils contiennent essentiellement des normales paraffines et des iso-paraffines, ces fluides présentant des coupes de distillation voisines et des caractéristiques proches de celles des fluides d'origine pétrolière. Ils sont dépourvus d'hydrocarbures aromatiques et ne contiennent pas de composés soufrés.

D'autres fluides spéciaux peuvent être obtenus à partir de la conversion du gaz naturel et/ou du charbon par transformation en gaz de synthèse puis selon le procédé Fischer Tropsch en composés hydrocarbonés susceptibles d'être séparés en coupes de distillation de caractéristiques comparables aux fluides spéciaux décrits précédemment, c'est-à-dire sans soufre et comprenant essentiellement des normales paraffines et des isoparaffines.

Dans l'industrie de la construction, de nombreux fluides spéciaux sont utilisés dans les matériaux de construction, par exemple pour les revêtements de sols, les peintures, les papiers peints et les mastics pour les fenêtres ou les joints de sanitaires, et les adhésifs de tous types. Ils sont constitués en général d'un ou deux composants actifs dont la viscosité est ajustée pour l'application visée par l'ajout d'un diluant hydrocarboné d'origine fossile c'est-à-dire principalement pétrolière faisant partie des fluides spéciaux. Ces diluants sont mélangés à au moins une résine, un polymère et/ou toute autre pâte de viscosité élevée et ont tendance soit tout de suite, soit avec le temps à s'évaporer et/ou se dégrader et à être une source d'émissions souvent toxiques pour l'environnement et plus particulièrement pour les santés humaine et animale. Ces émissions sont appelées des émissions de COV (ou Composés Organiques Volatils - VOC en anglais). Ces émissions constituent une source importante de pollution intérieure des habitations, des bureaux et des locaux et d'une manière générale tout espace fermé dont l'aération est limitée, en tout cas non ouverte directement à la circulation d'air.

Les fluides spéciaux sont également beaucoup utilisés notamment dans les fluides de forage. Dans cette application, on recherche particulièrement des fluides résistants aux conditions de températures et de pression très élevées, voire extrêmes, notamment celles rencontrées pour le forage de puits dans les off-shore profonds à plus de 4000 mètres sous le niveau de la mer ou dans les zones polaires ou proches de celles-ci. En effet, lorsqu'on opère dans les grands fonds marins jusqu'à 5500 m, le gradient de température entre l'entrée du puits et le fond du puits peut atteindre 200°C, la température d'entrée du puits pouvant avoisiner des températures polaires et la température de fond de puits plus de 160°C. Les fluides de forage entrent dans la composition des boues de forage à hauteur de 30 à 95% en poids. Ces boues de forage jouent un rôle essentiel lors des opérations de forage on shore ou off-shore, car elles permettent de lubrifier l'outil de forage (ou trépan) pour limiter son usure, mais aussi de remonter à la surface pour traitement, les déblais de roche (cuttings) générés lors du forage et de les maintenir en suspension lors des phases d'arrêt de la circulation de la boue, et enfin d'assurer le maintien de la pression dans la formation rocheuse afin d'éviter les fuites et/ou les effondrements de parois. Pour cette application, la maitrise de la viscosité cinématique, du point éclair, et du point d'écoulement est indispensable.

Dans les applications encres, on utilise traditionnellement trois principaux types d'impressions qui vont nécessiter l'emploi d'encres de types différents : l'impression en relief, l'impression en forme plate (ou impression en offset ou lithographie) et l'impression en héliogravure. Dans le domaine des encres offset, en fonction du type de séchage, on peut distinguer : les encres dites heatset pour rotatives à bobines qui sèchent par application de chaleur, les encres pour machines à feuilles dites sheetfed séchant par absorption et oxydation, et encore les encres coldset (encres à journaux) qui sèchent par absorption dans le substrat poreux.

Pour chacune de ces applications, la composition des encres est essentielle pour l'obtention du résultat. Les encres d'impression se composent de pigments, de liants, de solvants et d'additifs, mais leur répartition permet d'atteindre les propriétés désirées des encres pour chacune des impressions envisagées. Les diverses exigences auxquelles les propriétés physiques doivent répondre tout en tenant compte des critères économiques, en particulier dans les produits d'impression de masse, imposent des contraintes élevées aux solvants employés. D'une part, le solvant doit être capable de dissoudre les liants ainsi que les divers additifs (pouvoir solvant contrôlé), et d'autre part il doit permettre d'atteindre la viscosité et le tack dans l'intervalle désiré. Dans ces applications, les produits utilisés doivent présenter des caractéristiques d'évaporation optimales et un taux d'émission de COV les plus faibles possibles.

Dans une application en tant que lubrifiant, ces fluides peuvent être particulièrement efficaces pour le travail des métaux, comme fluide de protection contre l'oxydation ou encore l'électroérosion ou lors du laminage de l'aluminium. Ils ont pour fonction de minimiser les pressions et de dissiper la chaleur dans le travail des métaux. Ils permettent de diminuer la friction du fait de leurs propriétés lubrifiantes, de nettoyer les pièces, de limiter leur usure et de les protéger contre la corrosion, et de prolonger la durée de vie des outils.

Plus généralement les fluides spéciaux, du fait de leurs caractéristiques, sont liquides, inodores, très purs car de teneur en soufre réduite, et sont également dépourvus de substances toxiques, notamment de composés aromatiques mono et polycycliques. Ils sont particulièrement adaptés pour des applications nécessitant un temps de séchage court et une tension de surface adaptée car d'intervalle de coupe faible. Le niveau de pureté permet également de les utiliser dans la pharmacie par exemple dans les excipients pharmaceutiques.

La présente invention vise à remédier au problème de disponibilité de fluides spéciaux d'origine naturelle présentant des caractéristiques voisines à celles de fluides spéciaux actuellement utilisés et disponibles sur le marché. Elle vise en outre l'utilisation de ces nouveaux fluides spéciaux pour améliorer certaines des propriétés des fluides spéciaux classiques, notamment les propriétés à froid et la lubrifiance pour des applications à basses températures, voire grands froids.

### RESUME DE L'INVENTION

Le but de la présente invention est d'utiliser des composés d'origine naturelle et renouvelable, non toxiques et quasi purs, de caractéristiques physicochimiques proches de celles des fluides spéciaux, dont la stabilité et la viscosité mais aussi de taux de volatilité permet de les utiliser dans les mêmes applications que celles destinées aux fluides spéciaux, éventuellement dans des environnements ou des conditions d'utilisation plus difficiles.

La présente invention a donc pour objet une composition de fluide spécial comprenant au moins un poly-terpène hydrogéné choisi parmi les sesqui, di, ses-terpènes hydrogénés en mélange avec au moins un fluide spécial, ledit fluide spécial étant :<insérer p.5a>. L'invention concerne plus particulièrement une composition comprenant des polyterpènes hydrogénés, c'est-à-dire complètement saturés, composés de 3 à 5 motifs isoprènoïques sous forme linéaire et/ou cyclique choisis parmi les sesqui-, di- et ses-terpènes hydrogénés. Les poly-terpènes sont choisis en particulier parmi le farnesane, le cyclofarnésane, le bisabolane, le phytane et le labdane.
a) un mélange hydrocarboné d'origine pétrolière de température de coupe comprise entre 100 et 400°C, la température de coupe étant déterminée selon la norme ASTM D86 pour un point final de distillation inférieur à 365°C ou selon la norme ASTM D1160 pour un point final de distillation supérieur à 365°C, dont l'intervalle de distillation est inférieur à 75°C, ou
b) un mélange hydrocarboné d'origine synthétique de température de coupe comprise entre 100 et 400°C, la température de coupe étant déterminée selon la norme ASTM D86 pour un point final de distillation inférieur à 365°C ou selon la norme ASTM D1160 pour un point final de distillation supérieur à 365°C, dont l'intervalle de distillation est inférieur à 75°C, issu de la polymérisation et/ou de l'oligomérisation d'oléfines de 2 à 4 carbones, cette polymérisation conduisant à des produits comprenant de 2 à 5 motifs oléfiniques par chaîne, et contenant des normales paraffines et des isoparaffines, ou
c) obtenu à partir de la conversion du gaz naturel et/ou du charbon par transformation en gaz de synthèse puis selon le procédé Fischer Tropsch en composés hydrocarbonés susceptibles d'être après hydrogénation séparés en coupes de distillation de coupes comprises entre 100 et 400°C, la température de coupe étant déterminée selon la norme ASTM D86 pour un point final de distillation inférieur à 365°C ou selon la norme ASTM D1160 pour un point final de distillation supérieur à 365°C, et d'intervalle de distillation inférieur à 75°C, la teneur en soufre étant inférieure ou égale à 10ppm et la teneur en aromatique inférieure à 100ppm.

Plus particulièrement, la composition comprend au moins un poly-terpène hydrogéné choisi parmi les sesqui, di, ses-terpènes hydrogénés, pris seul ou en combinaison avec au moins un autre mono ou poly-terpène hydrogéné dans au moins un fluide spécial compatible en température d'ébullition, point éclair et densité. Le terme « compatible » signifie que ledit au moins un mono ou poly-terpène hydrogéné présente une température d'ébullition, un point éclair et une densité proches de ceux du ou des fluides spéciaux.

Le fluide spécial selon l'invention peut être obtenu par hydrogénation de coupes de distillation variant de 100 à 400°C, elles-mêmes obtenues par hydrocraquage, par hydrotraitement, par craquage catalytique, par cokage, par viscoréduction et/ou par hydrodéparaffinage, et distillation de ladite coupe, en coupes d'intervalle de distillation inférieur à 75°C. Ces fluides spéciaux sont constitués majoritairement, c'est-à-dire à plus de 50% en poids d'isoparaffines et de naphtènes, ont une teneur en soufre inférieure à 10 ppm et une teneur en aromatiques inférieure à 100 ppm. De préférence, la teneur en naphtènes de ces fluides est supérieure à 40% en poids, dont plus de 20% poids est composé de polynaphtènes.

Le fluide spécial peut également être issu de la polymérisation et/ou de l'oligomérisation d'oléfines de 2 à 4 carbones, cette polymérisation conduisant à des produits comprenant de 2 à 5 motifs oléfiniques par chaîne. Ils contiennent essentiellement des normales paraffines et des iso-paraffines.

Un autre fluide spécial peut être obtenu à partir de la conversion du gaz naturel et/ou du charbon par transformation en gaz de synthèse puis selon le procédé Fischer Tropsch en composés hydrocarbonés susceptibles d'être après hydrogénation séparés en coupes de distillation de coupes comprises entre 100 et 400°C et d'intervalle de distillation inférieur à 75°C, la teneur en soufre étant inférieure ou égale à 10 ppm et la teneur en aromatique inférieure à 100ppm.

La composition peut en outre comprendre au moins un monoterpène hydrogéné choisi parmi le p-menthane, le cis-pinane, le trans-pinane seul ou en combinaison avec au moins un fluide spécial de température de coupe comprise entre 160 et 250°C, de densité comprise entre 750 et 870 kg/m³, de point éclair inférieur à 100°C, mais supérieur à 30°C.

Selon un premier mode de réalisation, la composition comprend au moins un sesquiterpène hydrogéné tel que le farnésane, le cyclofarnésane, le bisabolane, en combinaison avec au moins un fluide spécial de température de coupe comprise entre 230 et 290°C, de densité comprise entre 760 et 820 kg/m³, de point éclair supérieur à 100°C.

Selon un deuxième mode de réalisation, la composition comprend au moins un diterpène choisi parmi le phytane, et le labdane en combinaison avec au moins un fluide spécial de température de coupe comprise entre 300 et 350°C, de densité comprise entre 780 et 830 kg/m³, de point éclair supérieur à 130°C.

Selon un troisième mode de réalisation, la composition comprend au moins un diterpène hydrogéné du groupe comprenant le phytane, et le labdane en combinaison avec au moins un fluide spécial de température de coupe supérieure à 300°C, de préférence comprise entre 350 et 400 °C, de densité comprise entre 790 et 840 kg/m³, de point éclair supérieur à 130 °C et de viscosité variant de 6,5 à 11 mm²/s.

Un deuxième objet de l'invention est l'utilisation de la composition de fluide spécial pour des applications industrielles comme l'industrie pétrolière, la construction comme les mastics et les peintures, les adhésifs, l'industrie des encres, le travail des métaux, le traitement et la protection des métaux, mais aussi pour des usages domestiques, dans l'agroalimentaire, l'automobile, l'industrie sanitaire, la pharmacie, les lubrifiants industriels, les combustibles domestiques, les applications de fluides autorisés pour les contacts alimentaires et la cosmétique.

Plus particulièrement, l'utilisation selon la présente invention correspond à différentes applications, notamment :
- dans les encres, comme fluide de forage, le travail de l'aluminium ou comme agent de nettoyage/dégraissage pour les compositions contenant des sesquiterpènes hydrogénés, notamment du farnésane,
- dans les encres d'imprimerie, comme lubrifiants, comme fluide de forage, en pharmacie, en cosmétique, dans les mastics, ou dans les produits phytosanitaires, et pour le traitement des textiles et des métaux pour les compositions contenant des di-terpènes hydrogénés,
- et enfin comme lubrifiants, pour la production des excipients pharmaceutiques et pour le traitement des textiles et des métaux, et également pour les applications dans les mastics pour les compositions contenant au moins un di-terpène hydrogéné, en mélange avec au moins un fluide spécial de températures de coupe supérieure à 350°C.

### DESCRIPTION DETAILLEE

Les terpènes hydrogénés introduits dans la composition selon l'invention sont obtenus par hydrogénation des terpènes issus de nombreuses plantes, en particulier des conifères. Ce sont des composants majeurs des résines naturelles et des essences produites à partir de ces résines comme dans l'essence de térébenthine. Ils peuvent aussi être produits par tout autre procédé de biosynthèse à partir de sucres ou de la biomasse. Ces terpènes hydrogénés constituent une nouvelle source de base pour les fluides spéciaux d'autant qu'ils sont liquides à température ambiante, et qu'ils sont obtenus à partir de substances naturelles renouvelables, qu'ils ne contiennent pas de soufre et moins de 100ppm de composés aromatiques. Ils sont particulièrement avantageux en ce qu'ils peuvent être produits avec un haut degré de pureté, par exemple plus de 95% de pureté nécessaire pour certaines applications envisagées. Ils présentent des caractéristiques très précises en termes de température d'ébullition, de point d'écoulement très bas, de capacité de séchage rapide (pour certains d'entre eux), d'absence de soufre et de composant toxique, de viscosité adaptée aux applications visées et enfin ils sont typiquement non émetteurs de COV (conformément au schéma de réduction des émissions de type AgBB (Allemagne), aux standards de qualité ou aux écolabels tels que GEV Emicode, Ange Bleu, US Greenguard...) et donc peu toxiques pour l'environnement et leur manipulation par des travailleurs. Sans composés aromatiques, ils sont peu toxiques.

Pour mettre en oeuvre l'invention, ces terpènes hydrogénés comprendront avantageusement de 3 à 5 motifs isoprènoïques sous forme linéaire et/ou cyclique. En effet, l'hydrogénation de composés isoprènoïques peut conduire à la formation de composés linéaires branchés et des composés cycliques, ces composés pouvant être obtenus seuls ou en mélange dans le produit hydrogéné final.

On choisira d'hydrogéner des poly-terpènes choisis parmi les sesqui-, di- et ses-terpènes, les produits hydrogénés préférés correspondant aux composés suivants : le farnesane, le cyclofarnésane, le bisabolane, le phytane, et le labdane.

Pour hydrogéner ces terpènes, on peut utiliser tout type d'hydrogénation connu de l'homme du métier, par exemple sous pression de 30 à 80 bars, à une température comprise entre 130 et 250°C en présence d'un catalyseur d'hydrogénation composé d'au moins un métal du groupe VIII tel que le nickel, le cobalt, le molybdène, le tungstène, le platine et/ou le palladium supporté par au moins un oxyde métallique du groupe constitué par la silice, l'alumine, la zircone et/ou l'oxyde de titane, cristallins ou amorphes ou encore une zéolithe.

Ces terpènes hydrogénés peuvent être utilisés en mélange avec des fluides spéciaux traditionnels tels que ceux issus des gammes Isanes, Ketrul, Spirdane, Hydroseal, Scriptane, Eolane et Gemseal de TOTAL FLUIDES ou encore comme des gammes ISOPAR d'Exxon, Shellsol de Shell, les Nexbase de Neste Oil, l'IP clean et IP solvant d'Idemitsu Kosan ou encore le SK Isol G de SK solvent comprenant essentiellement des isoparaffines et/ou des naphtènes.

L'ajout de ces terpènes hydrogénés aux fluides spéciaux traditionnels peut permettre notamment un ajustement des propriétés à froid, notamment de diminuer le point d'écoulement dudit fluide, d'ajuster son pouvoir solvant et donc son point d'aniline, ou encore de limiter les émissions COV liées à la dilution de certains composés émettant plus de COV. On peut également ajuster la densité, et/ou la viscosité d'une composition. Ainsi des fluides spéciaux proposés actuellement pour d'autres applications peuvent être utilisés dans de nouvelles applications du fait de l'introduction de terpènes hydrogénés appropriés en mélange avec ceux-ci.

Plus particulièrement, la composition peut comprendre au moins un poly-terpène hydrogéné, en combinaison avec au moins un autre mono ou poly-terpène hydrogéné, et au moins un fluide spécial compatible en température d'ébullition, point éclair et densité.

Ainsi, ces terpènes hydrogénés en mélange, permettent d'ajuster les caractéristiques d'au moins un fluide spécial de température de coupe comprise entre 100 et 400°C, dont l'intervalle de température de la dite coupe est inférieure à 75°C.

Ainsi selon un mode de mise en oeuvre de l'invention, une composition utilisable dans les résines, les peintures, les vernis, les adhésifs, les mastics, notamment les mastics silicones, silanes modifiés, polyuréthane et/ou acryliques, ou pour le dégraissage/nettoyage des parois, comprend au moins un terpène hydrogéné du groupe constitué par les sesquiterpènes hydrogénés tels que le farnésane, le cyclofarnésane, ou encore le bisabolane. Pour ces applications, ces terpènes hydrogénés peuvent être mélangés à au moins un fluide spécial de température de coupe comprise entre 180 et 290°C, de densité comprise entre 750 et 820 kg/m³, et de viscosité à 40°C déterminée par la norme ASTM D445 inférieure à 4mm²/s. Parmi les fluides spéciaux traditionnellement utilisés pour ces applications, ces terpènes hydrogénés seront facilement mis en mélange avec des SPIRDANE D40, D60, des ISANES IP140, IP175, IP185, ou IP200 ou encore EDC 99DW et EDC 95-11 vendus par Total Fluides. Parmi d'autres fluides spéciaux utilisables en mélange, on peut citer plus particulièrement les ISOPAR d'EXXONMOBIL CHEMICAL, le SOFTROL 100 de CHEVRON PHILLIPS Chemical ou encore la gamme Shellsol de Shell.

Pour des applications encres d'imprimerie, selon la viscosité souhaitée, les compositions selon l'invention comprennent des sesqui, di ou ses-terpènes hydrogénés en mélange avec au moins un fluide spécial adapté à ladite application. Ainsi, une composition selon l'invention peut comprendre du farnesane en mélange avec des fluides spéciaux de températures d'ébullition comprises entre 230 et 360°C, de densité comprise entre 760 et 820 kg/m³, de point éclair supérieur à 100°C, et dont la viscosité mesurée à 40°C est comprise entre 2 et 6,5 mm²/s. C'est ainsi que ces terpènes hydrogènés peuvent être utilisés en mélange avec des produits commerciaux, comme ceux de la gamme Scriptane de Total Fluides, mais aussi d'autres produits commerciaux du marché.

Une autre composition adaptée aux applications telles que lubrifiants pour le travail des métaux, mastics et adhésifs, solvant de dilution de polymères, en phytosanitaire, en pharmacie, en cosmétique, dans les encres d'imprimeries comprend du phytane, et/ou du labdane en mélange avec un fluide spécial de température de coupe comprise entre 230 et 360°C, de densité comprise entre 790 et 830 kg/m³, de point éclair supérieur à 130°C et de viscosité à 40°C comprise entre 3 et 11 mm²/s. Ladite composition selon l'invention peut également comprendre en mélange avec l'un au moins des composés phytane, labdane des coupes de type HYDROSEAL G232H, HYDROSEAL G240H, HYDROSEAL G250H, HYDROSEAL G270H, HYDROSEAL G3H, HYDROSEAL G400H, HYDROSEAL
G290H, HYDROSEAL G340H, SCRIPTANE PW24/27H, Scriptane PW25/28H, Scriptane PW26/29H, Scriptane PW28/32H, Scriptane PW30/35H vendus par Total Fluides. Parmi les autres fluides spéciaux utilisables, on trouve les EXXSOL D110, D120, D130 et D140 vendus par EXXONMOBIL CHEMICAL, les CONOSOL C200 et C260 de CONOCO PHILLIPS, et le Calumet LVP200 de Calumet, les Poliot 261 et LVP200 de Petrochem Carless, ou encore les YK2831 et YKD 130 de SK.

Une troisième composition, de température d'ébullition supérieure à 350°C comprendra avantageusement au moins un sesqui et/ou un ses terpène hydrogéné en mélange avec un fluide spécial de température de coupe supérieure à 280°C, de point éclair supérieur à 140°C, et de viscosité mesurée à 40°C supérieure à 7mm²/s. Cette composition peut être utilisée pour la production des excipients pharmaceutiques. En mélangeant un di-terpène hydrogéné avec l'HYDROSEAL G340H, on obtient des compositions à faibles émissions de COV (conformément au schéma de réduction des émissions de type AgBB (Allemagne), aux standards de qualité ou aux écolabels tels que GEV Emicode, Ange Bleu, US Greenguard...), ces compositions étant aussi utilisables dans des applications autres, telles que les matériaux de construction, et les matériaux pour l'automobile. Ces compositions peuvent être également utilisées avantageusement dans des mélanges d'élastomères, dans les lubrifiants ou dans les fluides pour le travail des métaux, notamment le travail de l'aluminium. Elles sont utilisables également dans les applications phytosanitaires.

Pour des applications forage, le farnésane sera préféré parmi les terpènes hydrogénés utilisés selon l'invention. Il pourra être utilisé en mélange avec des fluides spéciaux de température de coupe comprise entre 230 et 290°C, de densité comprise entre 760 et 820 kg/m³, de point éclair supérieur à 100°C. Parmi ceux-ci, les EDC 99DW, EDC 95-11, EDC Diamond ou EDC PEARL vendus par Total Fluides sont préférentiellement pris en mélange avec le farnésane, les viscosités pouvant être ajustées entre 2 et 5 mm²/s dans certaines applications.

Pour les applications phytosanitaires, les diterpènes hydrogénés comprenant le phytane, le labdane, peuvent être utilisés en combinaison, en mélange avec au moins un fluide spécial de température de coupe comprise entre 300 et 400°C, de densité comprise entre 800 et 830 kg/m³, de point éclair supérieur à 130 et de viscosité à 40°C supérieure à 4, de préférence supérieure à 7, mais inférieure à 15 mm²/s. Parmi les fluides phytosanitaires accessibles sur le marché, ces terpènes hydrogénés peuvent être utilisés en mélange avec du GENERA, BANOLE, du CITROLE, OVISPRAY, CATANE et FINAVESTAN, vendus pour la protection des fruits et légumes par épandage sur les cultures pendant leur croissance, ces produits étant vendus par Total Fluides.

Les fluides spéciaux pris en mélange avec l'un des terpènes hydrogénés présenteront de préférence un intervalle de coupe de distillation le plus faible possible, de préférence inférieur à 75°C et de préférence inférieur à 50°C pour obtenir toutes les qualités requises pour les applications visées.

Pour le travail des métaux et/ou applications textiles, on préfèrera des mélanges avec des HYDROSEAL G232H, G250H, G3H, G290H, G340H et G400H, ou encore des LUBRILAM S40L ou encore LUBRILAM S50L pour le laminage de l'aluminium.

Les exemples ci-après visent à illustrer l'invention et ne peuvent être utilisés en vue d'en limiter la portée.

### EXEMPLE 1

Le présent exemple vise à comparer les propriétés du farnésane obtenu par hydrogénation du farnesène, sesquiterpène hydrogéné avec celles des fluides spéciaux communément utilisés pour diverses applications et les mélanges possibles du farnésane avec d'autres fluides spéciaux.

Dans le tableau I ci-dessous, sont réunis les caractéristiques physicochimiques du farnésane et des fluides spéciaux commerciaux ainsi qu'un mélange 50/50 farnésane / fluide spécial pour une application forage.

**TABLEAU I**

| Propriétés | Unités | Méthode | Farnésane | EDC99DW | Ketrul D80 | Ketrul/Farnésane 50/50 pds |
|---|---|---|---|---|---|---|
| Aspect | - | | **limpide** | limpide | limpide | limpide |
| Densité à 15°C | kg/m³ | NF EN ISO 12185 | **774** | 811 | 811 | 791,97 |
| Viscosité à 40°C | mm²/s | EN ISO 3104 | **2,5** | 2,30 | 1,70 | 1,96 |
| Point écoulement | °C | T 60 105 | **< - 71** | -51 | -51 | -67 |
| Point éclair | °C | EN ISO 2719 | **103,0** | 101 | 76 | 85 |
| Teneur en Aromatique | ppm | UV | **0** | 25 | <20 | <20 |
| Soufre total | ppm | FX/D2622 | - | <1 | <1 | <1 |

| **Distillation** | | | | | | |
|---|---|---|---|---|---|---|
| Point initial | °C | EN ISO 3405 | **242** | 230 | 201 | 201 |
| Point final | °C | EN ISO 3405 | **271** | 270 | 239 | 270 |

Pour l'application forage, il est bien connu d'utiliser l'EDC99DW qui présente les meilleures caractéristiques.

On constate que les propriétés du farnésane, en particulier sa viscosité, son point d'écoulement, sa température d'ébullition et son point éclair font de ce produit un bon fluide de forage. Par contre, le Ketrul D80 inutilisable dans cette application du fait de son point éclair trop bas, peut le devenir en mélange à 50/50 en poids avec du farnésane : le point éclair devient supérieur à 80°C ce qui permet d'atteindre un bon niveau de sécurité avec ce mélange et un moyen de pallier les déficits en EDC99DW notamment. Le farnésane améliore également le point d'écoulement qui rend ce mélange particulièrement efficace dans les zones froides.

Comme solvant dans les encres, la gamme Scriptane de Total fluides est particulièrement adaptée : le tableau II ci-après compare les propriétés du Scriptane 25/28 utilisé pour certaines applications encres avec celles d'un Scriptane 24/27H inapte à cette application, et avec celles du farnésane.

**TABLEAU II**

| Propriétés | Unités | Méthode | Farnesane | Scriptane 25/28H | Scriptane 24/27H | Scriptane 24/27H/Farnesane 50/50 pds |
|---|---|---|---|---|---|---|
| Aspect | - | | **limpide** | limpide | limpide | limpide |
| Densité à 15°C | kg/m3 | NF EN ISO 12185 | **774** | 815 | 817 | 793,97 |
| Viscosité à 40°C | mm²/s | EN ISO 3104 | **2,5** | 2,8 | 2,4 | 2,3 |
| Point écoulement | °C | T 60 105 | **< -71** | -40 | -50 | -66 |
| Point éclair | °C | EN ISO 2719 | **103,0** | 114 | 103 | 103 |
| Point d'aniline | °C | EN ISO 2977 | **>87** | 87 | 79 | 84 |
| Teneur en Aromatique | ppm | UV | **0** | 50 | 50 | 50 |
| Soufre total | ppm | FX/D2622 | - | <1 | <1 | <1 |

| **Distillation** | | | | | | |
|---|---|---|---|---|---|---|
| Point initial | °C | EN ISO 3405 | **242** | 251 | 237 | 237 |
| Point final | °C | EN ISO 3405 | **271** | 281 | 262 | 271 |

On constate que le farnésane permet d'atteindre des caractéristiques du Scriptane 25/28H avec une coupe plus étroite : la viscosité est comparable pour un meilleur point d'écoulement.

Pour le travail des métaux, notamment le laminage de l'aluminium, il est usuel d'utiliser des lubrifiants appelés Lubrilam. Le tableau III réunit les caractéristiques physicochimiques de deux fluides lubrilam et du farnésane seul et en mélange 50/50 en poids avec le lubrilam S50L.

**TABLEAU III**

| Propriétés | Unités | Méthode | Farnesane | LUBRILAM S40L | LUBRILAM S50L | LUBRILAM S50L/Farnesane 50/50 pds |
|---|---|---|---|---|---|---|
| Aspect | - | | **limpide** | limpide | limpide | limpide |
| Densité à 15°C | kg/m3 | NF EN ISO 12185 | **774** | 820 | 817 | 794,97 |
| Viscosité à 40°C | mm²/ s | EN ISO 3104 | **2,5** | 2,4 | 2,8 | 2,4 |
| Point écoulement | °C | T 60 105 | **<-71** | -50 | -40 | <-70 |
| Point éclair | °C | EN ISO 2719 | **103,0** | 103 | 116 | 111 |
| Teneur en Aromatique | ppm | UV | 0 | 30 | 30 | <30 |
| Soufre total | ppm | FX/D262 2 | **<2** | <2 | <2 | <2 |

| **Distillation** | | | | | | |
|---|---|---|---|---|---|---|
| Point initial | °C | EN ISO 3405 | **242** | 236 | 254 | 245 |
| Point final | °C | EN ISO 3405 | **271** | 262 | 282 | 270 |

On constate que le farnésane peut se substituer seul au Lubrilam S40L. Le mélange 50/50 en poids de farnésane avec du Lubrilam S50L peut également convenir.

### EXEMPLE 2 (ne faisant pas partie de l'invention)

Le présent exemple concerne l'application aux peintures des compositions comprenant du pinane et présentant un pouvoir solvant important sans présenter les inconvénients des solvants aromatiques classiques.

Idéalement, un solvant hautement aromatique, avec un point d'aniline bas et une coupe de distillation étroite est préféré pour assurer un bon pouvoir solvant et un temps de séchage contrôlé. C'est ainsi que le solvarex 9 de Total fluides, correspondant à une coupe aromatique en C9 (comprenant 9 carbones) présentant un point d'aniline de 14 et une coupe de distillation variant de 160 à 175°C, est particulièrement apprécié. Cependant, du fait des restrictions réglementaires d'utilisation de produits fortement aromatiques, il a été remplacé par des white spirit déaromatisés dans les compositions de peintures décoratives et industrielles, ces white spirit présentant un point éclair de 40°C, comme par exemple le Spirdane D40 dont le point d'aniline est de 68 et la coupe de distillation comprise entre 156 et 198°C.

Dans le tableau IV ci-après sont données les caractéristiques des compositions de fluides spéciaux, contenant des composés aromatiques, des composés déaromatisés et les mélanges 50/50 Pinane composés déaromatisés.

**TABLEAU IV**

| Propriétés | Unités | Méthode | Pinane | Spirdane D40/Pinane 50/50 | Solvarex 9/fluide aromatique en C9 | Spirdane D40/white spirit déaromatisé |
|---|---|---|---|---|---|---|
| Aspect | - | | Clair & limpide | Clair& limpide | Clair& limpide | Clair& limpide |
| Densité à 15°C | kg/m3 | NF EN ISO 12185 | 861,4 | 818,2 | 875 | 775 |
| Couleur Saybolt | | NF M 07003 | >+30 | >+30 | >+30 | >+30 |
| Viscosité à 20°C | mm²/ s | EN ISO 3104 | 2,655 | 1,7 | 0,95 | 1,1 |
| Viscosité à 40°C | mm²/ s | EN ISO 3104 | 2,5 | 2,4 | 2,8 | 2,4 |
| Point d'aniline | | ASTM D611 | 40,7 | 55 | 14 | 68 |
| Point écoulement | °C | T 60 105 | <-72 | <-50 | <-50 | <-50 |
| Point éclair | °C | EN ISO 2719 | 45 | 44 | 43 | 44 |
| Teneur en Aromatique | ppm | UV | 0 | 0 | 99,5 | 0 |

| **Distillation** | | | | | | |
|---|---|---|---|---|---|---|
| Point initial | °C | EN ISO 3405 | 164.5 | 160 | 160 | 156 |
| Point final | °C | EN ISO 3405 | 165,5 | 188 | 175 | 198 |

On constate que le pinane utilisé en mélange avec du white spirit déaromatisé améliore le pouvoir solvant de ces white spirit (passage du point d'aniline de 68 à 55) et diminue la largeur de coupe de ceux-ci. En outre, le pinane permet d'améliorer la volatilité et le temps de séchage des formulations est diminué. Bien entendu, il est possible d'ajuster le rapport pinane/ White spirit déaromatisé pour optimiser le temps de séchage.

### EXEMPLE 3 (ne faisant pas partie de l'invention)

Le présent exemple vise l'utilisation de pinane dans des compositions utilisées pour le nettoyage des outils industriels et les pièces métalliques. Il s'agit ici d'améliorer le contrôle de la volatilité des produits utilisés pour le nettoyage.

Généralement, on utilise des isoparaffines de type ISANE 155 vendu par Total fluides ou encore un ISOPAR vendu par EXXON Chemical qui présentent des intervalles de coupe de distillation étroites et donc un intervalle de température de séchage étroit, idéal pour procédé de séchage par flash(*). Cependant, ces isoparaffines, ISANES ou ISOPAR présentent un pouvoir solvant faible. Dans le tableau V ci-après sont données les caractéristiques de l'ISANE IP155, celles du pinane et celles d'un mélange 50/50 pinane/ISANE IP155.

(*) Le procédé flash permet d'éliminer le solvant en portant la surface nettoyée à une température adaptée pendant un temps très court pour vaporiser le solvant.

**TABLEAU V**

| Propriétés | Unités | Méthode | Pinane | ISANE IP155 | ISANE IP155/ pinane 50/50 |
|---|---|---|---|---|---|
| Aspect | | | Clair & limpide | Clair & limpide | Clair & limpide |
| Densité à 15°C | kg/m³ | NF EN ISO 12185 | 861,4 | 746 | 799 |
| Couleur Saybolt | | NF M 07003 | >+30 | >+30 | >+30 |
| Viscosité à 20°C | mm²/s | EN ISO 3104 | 2,655 | 1,2 | 2,1 |
| Viscosité à 40°C | mm²/s | EN ISO 3104 | 2,5 | 1 | 1,8 |
| Point d'aniline | | ASTM D611 | 40,7 | 78 | 61 |
| Point écoulement | °C | T 60 105 | < - 72 | <-50 | -62 |
| Teneur en Aromatique | ppm | UV | 0 | 0 | 0 |

| **Distillation** | | | | | |
|---|---|---|---|---|---|
| Point initial | °C | EN ISO 3405 | 164.5 | 158 | 160 |
| Point final | °C | EN ISO 3405 | 165.5 | 175 | 169 |

On constate d'après ce tableau que l'utilisation de pinane en mélange avec des isoparaffines ordinairement utilisées pour des nettoyages de pièces et/ou d'outils industriels permet d'améliorer nettement son pouvoir solvant qui est lié à la valeur du point d'aniline.

### EXEMPLE 4

Le présent exemple vise l'utilisation du phytane dans des compositions de l'invention notamment pour toutes les applications industrielles (mastics silicones et lubrifiants notamment) exigeant une bonne fluidité à basse température dans les conditions sévères d'utilisation ou de stockage. Le phytane peut être utilisé tel quel ou en mélange avec l'Hydroseal 310 H qui est un fluide hydrocarboné désaromatisé et isodéparaffiné.

**TABLEAU VI**

| Propriétés | Unités | Méthode | Phytane | Hydroseal G310H | Phytane Hydroseal G310 H 50/50 |
|---|---|---|---|---|---|
| Aspect | | | Clair & limpide | Clair & limpide | Clair & limpide |
| Densité à 15°C | kg/m3 | NF EN ISO 12185 | 791 | 818 | 809 |
| Couleur Saybolt | | NFM 07003 | - | < 0,5 | < 0,5 |
| Viscosité à 20°C | mm²/s | EN ISO 3104 | 12 | 10,9 | 11,4 |
| Viscosité à 40°C | mm²/s | EN ISO 3104 | 5,9 | 6,0 | 5,9 |
| Point d'aniline | | ASTM D611 | 92 | 99 | 98 |
| Point écoulement | °C | T 60 105 | < - 30 | <-18 | -27 |
| Teneur en Aromatique | ppm | UV | 0 | 0 | 0 |

| **Distillation** | | | | | |
|---|---|---|---|---|---|
| Point initial | °C | ASTM D86 | 322 | 300 | 300 |
| Point final | °C | ASTM D86 | 324 | 350 | 350 |

Les fluides à base de phytane sont particulièrement performants aux basses températures avec un point d'écoulement inférieur à - 24°C, ce qui est particulièrement souhaitable lorsqu'on opère aux basses températures ou que les produits finis sont maintenus à de telles températures pendant longtemps.

## Revendications

1. Composition de fluide spécial comprenant au moins un poly-terpène hydrogéné choisi parmi les sesqui, di, ses-terpènes hydrogénés, en mélange avec au moins un fluide spécial, ledit fluide spécial étant:
a) un mélange hydrocarboné d'origine pétrolière de température de coupe comprise entre 100 et 400°C, la température de coupe étant déterminée selon la norme ASTM D86 pour un point final de distillation inférieur à 365°C ou selon la norme ASTM D1160 pour un point final de distillation supérieur à 365°C, dont l'intervalle de distillation est inférieur à 75°C, ou
b) un mélange hydrocarboné d'origine synthétique de température de coupe comprise entre 100 et 400°C, la température de coupe étant déterminée selon la norme ASTM D86 pour un point final de distillation inférieur à 365°C ou selon la norme ASTM D1160 pour un point final de distillation supérieur à 365°C, dont l'intervalle de distillation est inférieur à 75°C, issu de la polymérisation et/ou de l'oligomérisation d'oléfines de 2 à 4 carbones, cette polymérisation conduisant à des produits comprenant de 2 à 5 motifs oléfiniques par chaîne, et contenant des normales paraffines et des isoparaffines, ou
c) obtenu à partir de la conversion du gaz naturel et/ou du charbon par transformation en gaz de synthèse puis selon le procédé Fischer Tropsch en composés hydrocarbonés susceptibles d'être après hydrogénation séparés en coupes de distillation de coupes comprises entre 100 et 400°C, la température de coupe étant déterminée selon la norme ASTM D86 pour un point final de distillation inférieur à 365°C ou selon la norme ASTM D1160 pour un point final de distillation supérieur à 365°C, et d'intervalle de distillation inférieur à 75°C, la teneur en soufre étant inférieure ou égale à 10ppm et la teneur en aromatique inférieure à 100ppm.

2. Composition selon la revendication 1, dans laquelle les poly-terpènes hydrogénés sont choisis parmi le farnésane, le cyclofarnésane, le bisabolane, le phytane, le labdane.

3. Composition selon l'une des revendications précédentes **caractérisée en ce que** le fluide spécial est obtenu par hydrogénation de coupes de distillation variant de 100 à 400°C, elles-mêmes obtenues par hydrocraquage, par hydrotraitement, par craquage catalytique, par cokage, par viscoréduction et/ou par hydrodéparaffinage, et distillation desdites coupes en coupes d'intervalle de distillation inférieur à 75°C, et comprenant moins de 10 ppm de soufre et moins de 100ppm de composés aromatiques.

4. Composition selon l'une des revendications 1 à 3, comprenant au moins un sesquiterpène tel que le farnésane, le cyclofarnésane ou le bisabolane, en combinaison avec au moins un fluide spécial de température de coupe comprise entre 230 et 290°C, de densité comprise entre 760 et 820 kg/m³, de point éclair supérieur à 100°C.

5. Composition selon l'une des revendications 1 à 3, comprenant au moins un diterpène choisi parmi le phytane, le labdane, en combinaison avec au moins un fluide spécial de température de coupe comprise entre 300 et 350°C, de densité comprise entre 800 et 830 kg/m³, de point éclair supérieur à 130°C.

6. Composition selon l'une des revendications 1 à 3, comprenant au moins un di-terpène hydrogéné du groupe comprenant le phytane, le labdane, en combinaison avec au moins un fluide spécial de température de coupe supérieure à 300°C, de densité comprise entre 790 et 840 kg/m³, de point éclair supérieur à 130°C et de viscosité variant de 6,5 à 11 mm²/s.

7. Utilisation de la composition selon l'une des revendications 1 à 6 pour des applications dans l'industrie pétrolière, la construction, les usages domestiques, l'automobile, l'agroalimentaire, la pharmacie, les lubrifiants industriels, les combustibles domestiques et les applications de fluides autorisés pour les contacts alimentaires.

8. Utilisation selon la revendication 7 dans les encres, comme fluide de forage, pour le travail de l'aluminium ou comme agent de nettoyage/dégraissage, ladite composition comprenant des sesquiterpènes hydrogénés, en particulier du farnesane.

9. Utilisation selon la revendication 7 dans les encres d'imprimerie, comme lubrifiants, comme fluide de forage, en pharmacie, dans les mastics, dans les produits phytosanitaires et pour le traitement des textiles et des métaux, ladite composition comprenant des di-terpènes hydrogénés.

10. Utilisation selon la revendication 7 comme lubrifiants, pour la production des excipients pharmaceutiques, pour le traitement des textiles et des métaux, et pour les applications pour mastics, ladite composition comprenant au moins un di-terpènes hydrogénés, en mélange avec au moins un fluide spécial de températures de coupe supérieures à 350°C.

## Patentansprüche

1. Spezielle Fluidzusammensetzung, umfassend mindestens ein hydriertes Polyterpen, ausgewählt aus hydrierten Sesqui-, Di-, Sesterpenen, in Mischung mit mindestens einem speziellen Fluid, wobei das spezielle Fluid ist:
a) eine aus Erdöl gewonnenen Kohlenwasserstoff-Mischung von auf Erdöl-Basis mit einer Schnitttemperatur zwischen 100 und 400°C, wobei die Schnitttemperatur gemäß der Norm ASTM D86 für einen Enddestillationspunkt unter 365°C, oder gemäß der Norm ASTM D1160 für einen Enddestillationspunkt über 365°C, bestimmt wird, wobei das Destillationsintervall kleiner ist als 75°C, oder
b) eine Kohlenwasserstoff-Mischung aus synthetischer Basis mit einer Schnitttemperatur zwischen 100 und 400°C, wobei die Schnitttemperatur gemäß der Norm ASTM D86 für einen Enddestillationspunkt unter 365°C, oder gemäß der Norm ASTM D1160 für einen Enddestillationspunkt über 365°C, bestimmt wird, wobei das Destillationsintervall kleiner ist als 75°C, aus der Polymerisation und/oder Oligomerisation von Olefinen mit 2 bis 4 Kohlenstoffatomen, wobei diese Polymerisation zu Produkten führt, die 2 bis 5 Olefin-Einheiten pro Kette umfassen, und normale Paraffine und Isoparaffine enthalten, oder
c) erhalten aus der Umwandlung von Erdgas und/oder von Kohle durch Transformation in Synthesegas, dann gemäß dem Fischer-Tropsch-Verfahren in KohlenwasserstoffVerbindungen, die nach einer Hydrierung in Destillationsschnitte von Schnitten zwischen 100 und 400°C getrennt werden können, wobei die Schnitttemperatur gemäß der Norm ASTM D86 für einen Enddestillationspunkt unter 365°C, oder gemäß der Norm ASTM D1160 für einen Enddestillationspunkt über 365°C, bestimmt wird, und wobei das Destillationsintervall kleiner ist als 75°C, wobei der Schwefelgehalt kleiner oder gleich 10 ppm ist, und der Aromatengehalt kleiner ist als 100 ppm.

2. Zusammensetzung nach Anspruch 1, wobei die hydrierten Polyterpene ausgewählt sind aus Farnesan, Cyclofarnesan, Bisabolan, Phytan, Labdan.

3. Zusammensetzung nach einem der vorhergehenden Ansprüche, **dadurch gekennzeichnet, dass** das spezielle Fluid erhalten wird durch Hydrierung von Destillationsschnitten variierend von 100 bis 400°C, wobei diese selbst erhalten werden durch Hydro-Kracken, durch Hydrobehandlung, durch katalytisches Kracken, durch Koken, durch Visbreaking und/oder durch Hydroentparaffinierung, und Destillation der Schnitte in Schnitte mit einem Destillationsintervall kleiner als 75°C, und umfassend weniger als 10 ppm Schwefel und weniger als 100 ppm aromatische Verbindungen.

4. Zusammensetzung nach einem der Ansprüche 1 bis 3, umfassend mindestens ein Sesquiterpen, wie Farnesan, Cyclofarnesan oder Bisabolan, in Kombination mit mindestens einem speziellen Fluid mit einer Schnitttemperatur zwischen 230 und 290°C, mit einer Dichte zwischen 760 und 820 kg/m³, mit einem Flammpunkt über 100°C.

5. Zusammensetzung nach einem der Ansprüche 1 bis 3, umfassend mindestens ein Diterpen, ausgewählt aus Phytan, Labdan, in Kombination mit mindestens einem speziellen Fluid mit einer Schnitttemperatur zwischen 300 und 350°C, mit einer Dichte zwischen 800 und 830 kg/m³, mit einem Flammpunkt über 130°C.

6. Zusammensetzung nach einem der Ansprüche 1 bis 3, umfassend mindestens ein hydriertes Diterpen der Gruppe umfassend Phytan, Labdan, in Kombination mit mindestens einem speziellen Fluid mit einer Schnitttemperatur über 300°C, mit einer Dichte zwischen 790 und 840 kg/m³, mit einem Flammpunkt über 130°C, und mit einer Viskosität variierend von 6,5 bis 11 mm²/s.

7. Verwendung der Zusammensetzung nach einem der Ansprüche 1 bis 6, für Anwendungen in der Erdölindustrie, dem Bauwesen, Haushaltsanwendungen, für Fahrzeuge, in der Nahrungsmittelindustrie, der Pharmazie, für industrielle Schmiermittel, Haushaltsbrennstoffe und Anwendungen von Fluiden, die für den Kontakt mit Nahrungsmitteln zugelassen sind.

8. Verwendung nach Anspruch 7, in Tinten, als Bohrfluid, für Aluminiumarbeiten oder als Reinigungs/Entfettungsmittel, wobei die Zusammensetzung hydrierte Sequiterpene, insbesondere Farnesan, umfasst.

9. Verwendung nach Anspruch 7, in Drucktinten, als Schmiermittel, als Bohrfluid, in der Pharmazie, in Spachtelmassen, in phytosanitären Produkten und zur Behandlung von Textilien und Metallen, wobei die Zusammensetzung hydrierte Diterpene umfasst.

10. Verwendung nach Anspruch 7, als Schmiermittel, zur Herstellung pharmazeutischer Exzipienten, zur Behandlung von Textilien und Metallen, und für Anwendungen für Spachtelmassen, wobei die Zusammensetzung mindestens ein hydriertes Diterpen umfasst, in Mischung mit mindestens einem speziellen Fluid mit Schnitttemperaturen über 350°C.

## Claims

1. Composition of special fluid comprising at least one hydrogenated polyterpene selected from hydrogenated sesqui, di, ses-terpenes, in mixture with at least one special fluid, said special fluid being:
a) a hydrocarbon mixture of petroleum origin with a cut temperature comprised between 100 and 400°C, the cut temperature being determined according to standard ASTM D86 for a final distillation point less than 365°C or according to ASTM D1160 for a final distillation point above 365°C, the distillation range being less than 75°C, or
b) a hydrocarbon mixture of synthetic origin with a cut temperature comprised between 100 and 400°C, the cut temperature being determined according to standard ASTM D86 for a final distillation point less than 365°C or according to ASTM D1160 for a final distillation point above 365°C, the distillation range being less than 75°C, originating from the polymerization and/or oligomerization of olefins with 2 to 4 carbon atoms, this polymerization leading to products comprising from 2 to 5 olefin units per chain, and containing normal paraffins and iso-paraffins, or
c) obtained from the conversion of natural gas and/or coal by conversion to synthesis gas, then according to the Fischer-Tropsch process, to hydrocarbon compounds capable of being separated, after hydrogenation, into distillation cuts with cuts comprised between 100 and 400°C, the cut temperature being determined according to standard ASTM D86 for a final distillation point less than 365°C or according to ASTM D1160 for a final distillation point above 365°C, the distillation range being less than 75°C, the sulphur content being less than or equal to 10 ppm and the aromatic content being less than 100 ppm.

2. Composition according to claim 1, in which the hydrogenated polyterpenes are selected from farnesane, cyclofarnesane, bisabolane, phytane, labdane.

3. Composition according to one of the preceding claims, **characterized in that** the special fluid is obtained by hydrogenation of distillation cuts varying from 100 to 400°C, themselves obtained by hydrocracking, by hydrotreatment, by catalytic cracking, by coking, by vis-breaking and/or by hydrodewaxing, and distillation of said cuts into cuts with a distillation range of less than 75°C, and comprising less than 10 ppm of sulphur and less than 100 ppm of aromatic compounds.

4. Composition according to one of claims 1 to 3, comprising at least one sesquiterpene such as farnesane, cyclofarnesane or bisabolane, in combination with at least one special fluid with a cut temperature comprised between 230 and 290°C, with a density comprised between 760 and 820 kg/m³, with a flash point above 100 °C.

5. Composition according to one of claims 1 to 3, comprising at least one diterpene selected from phytane, labdane in combination with at least one special fluid with a cut temperature comprised between 300 and 350°C, with a density comprised between 800 and 830 kg/m³, and a flash point above 130°C.

6. Composition according to one of claims 1 to 3, comprising at least one hydrogenated diterpene from the group comprising phytane, labdane in combination with at least one special fluid with a cut temperature above 300 °C, with a density comprised between 790 and 840 kg/m³, and a flash point above 130°C and a viscosity varying between 6.5 and 11 mm²/s.

7. Use of the composition according to one of claims 1 to 6, for applications in the petroleum industry, construction, domestic uses, the automobile, agric-food, pharmaceutical industries, for industrial lubricants, domestic fuels, and applications of fluids permitted for contact with food.

8. Use according to claim 7, in inks, as drilling fluid, for working aluminium or as a cleaning/degreasing agent, said composition comprising hydrogenated sesqui-terpenes, in particular farnesane.

9. Use according to claim 7, in printing inks, as lubricants, as drilling fluid, in the pharmaceuticals industry, in sealants, in phytosanitary products and for the treatment of textiles and metals, said composition comprising hydrogenated di-terpenes.

10. Use according to claim 7, as lubricants, for the production of pharmaceutical excipients, for the treatment of textiles and metals, and for applications for sealants, said composition comprising at least one hydrogenated di-terpene, in a mixture with at least one special fluid with a cut temperature above 350°C.
